# DEMANDE DE BREVET EUROPEEN

(11) **EP 2 374 753 A1**
(43) Date de publication de la demande: **12.10.2011**
(21) Numéro de dépôt: 11305355.7
(22) Date de dépôt: 30.03.2011
(51) Int. Cl.: C01B 13/11, H05B 6/80

(54) **Dispositif de production d'une espèce chimique à partir d'un fluide grâce à la mise en oeuvre d'une structure résonante micro-ondes**

(30) Priorité: 01.04.2010 FR 1052493
(71) Demandeur: Centre National de la Recherche Scientifique (C.N.R.S), 75016 Paris (FR)
(72) Inventeur: Pierre, Thiéry Luc Frédéric, 13013, MARSEILLE (FR)
(74) Mandataire: Jacobson, Claude

(57) **Abrégé**

Dispositif (4) de production d'une espèce chimique à partir d'un fluide (F) comprenant :
- une source de micro-ondes (30) ;
- une structure résonante micro-ondes (32) comportant un générateur de plasma ;
-des moyens (39) de transmission des micro-ondes de la source de micro-ondes vers la structure résonante micro-ondes ;
- une chambre de réaction (50) accueillant le générateur de plasma ;
- des moyens (10) d'introduction du fluide (F) dans la chambre de réaction ; et
- des moyens (12) d'extraction de l'espèce chimique de la chambre de réaction, la structure résonante micro-ondes comprenant un élément résonant, notamment filaire, de type antenne quart d'onde (52).

## Description

La présente invention concerne un dispositif de production d'une espèce chimique du type décrit dans le préambule de la revendication 1.

On connaît du document WO 2004/071953 A1 un dispositif de production d'une espèce chimique à partir d'un fluide comprenant une source de micro-ondes, une structure résonante micro-ondes comportant un générateur de plasma, des moyens de transmission des micro-ondes de la source de micro-ondes vers la structure résonante micro-ondes, une chambre de réaction accueillant le générateur de plasma, des moyens d'introduction du fluide dans la chambre de réaction et des moyens d'extraction de l'espèce chimique de la chambre de réaction.

Ce document décrit une unité de dissociation de molécules d'oxygène comprenant une source de micro-ondes, à savoir un magnétron, une cavité résonnante coaxiale et un guide micro-ondes reliant la cavité résonante au magnétron. Un plasma est créé au sein de la cavité résonante par application de micro-ondes d'une fréquence de 2,45 GHz. Du dioxygène est introduit dans la cavité résonante et dissocié par le plasma en atomes d'oxygène. Les atomes d'oxygène sont extraits de la cavité résonante et mis en contact avec du dioxygène excité afin de générer de l'ozone.

On connaît de US 2009/0134152 A1 un dispositif de production de plasma à partir d'un fluide comprenant une source micro-ondes, un élément résonant de type antenne quart d'onde et une chambre de réaction, l'antenne quart d'onde se trouvant en dehors de la chambre de réaction.

En outre, on connaît de US 2007/0051315 A1 un dispositif de production d'une nanopoudre à partir d'un fluide du type précité. US 2007/0051315 A1 est considéré comme étant l'état de la technique le plus proche du dispositif selon l'invention.

Néanmoins, on constate que ces dispositifs connus sont encombrants et consomment beaucoup d'énergie.

Un but de l'invention est donc de réaliser un dispositif de production d'une espèce chimique qui soit à la fois compact et économe en énergie. Dans le cas de la production d'ozone, un but est notamment la réalisation d'un générateur d'ozone fonctionnant efficacement sans séchage préalable de l'air introduit dans le générateur, contrairement aux générateur d'ozone existants basés sur les décharges de type couronne.

Selon l'invention, ces buts sont atteints par un dispositif du type précité, caractérisé par la partie caractérisante de la revendication 1.

En réalisant la structure résonante micro-ondes avec un élément de type antenne quart d'onde à l'intérieur de la chambre de réaction on obtient un dispositif de petite taille, puisque les micro-ondes ont des longueurs d'ondes inférieures à 1 mètre. Grâce à la structure précisée dans la partie caractérisante de la revendication 1, le dispositif selon l'invention requiert une puissance d'uniquement quelques watts et consomme donc peu d'énergie.

Selon d'autres modes de réalisation, le dispositif comporte une ou plusieurs des caractéristiques des revendications dépendantes 2 à 11 prise(s) isolément ou selon toutes les combinaisons techniquement possibles.

L'invention concerne également une installation de traitement par fluide en circuit fermé selon la revendication 12.

L'invention concerne aussi un procédé de production d'une espèce chimique à l'aide d'un dispositif tel que défini ci-dessus selon la revendication 13.

L'invention concerne en outre une utilisation d'un dispositif tel que décrit ci-dessus pour la production d'une espèce chimique à partir d'un fluide selon les revendications 14 et 15comprenant du dioxygène et un moyen apte à dissocier le dioxygène en atomes d'oxygène en présence de micro-ondes. Le fluide peut être de l'air et le moyen de dissociation est alors l'azote de l'air.

L'invention sera mieux comprise à la lecture de la description qui va suivre, donnée uniquement à titre d'exemple, et faite en se référant aux dessins annexés sur lesquels :
- la figure 1 est une vue d'ensemble d'une installation de traitement par fluide selon l'invention ;
- la figure 2 est une vue en coupe d'un dispositif de production d'une espèce chimique selon l'invention ;
- la figure 3 est une vue en perspective d'un premier mode de réalisation d'une structure résonante micro-ondes selon l'invention ;
- la figure 4 est une vue de côté de la structure résonante micro-ondes selon la figure 3 ;
- la figure 5 est une vue en perspective d'un deuxième mode de réalisation d'une structure résonante micro-ondes selon l'invention ; et
- la figure 6 est une vue en perspective d'un troisième mode de réalisation d'une structure résonante micro-ondes selon l'invention.

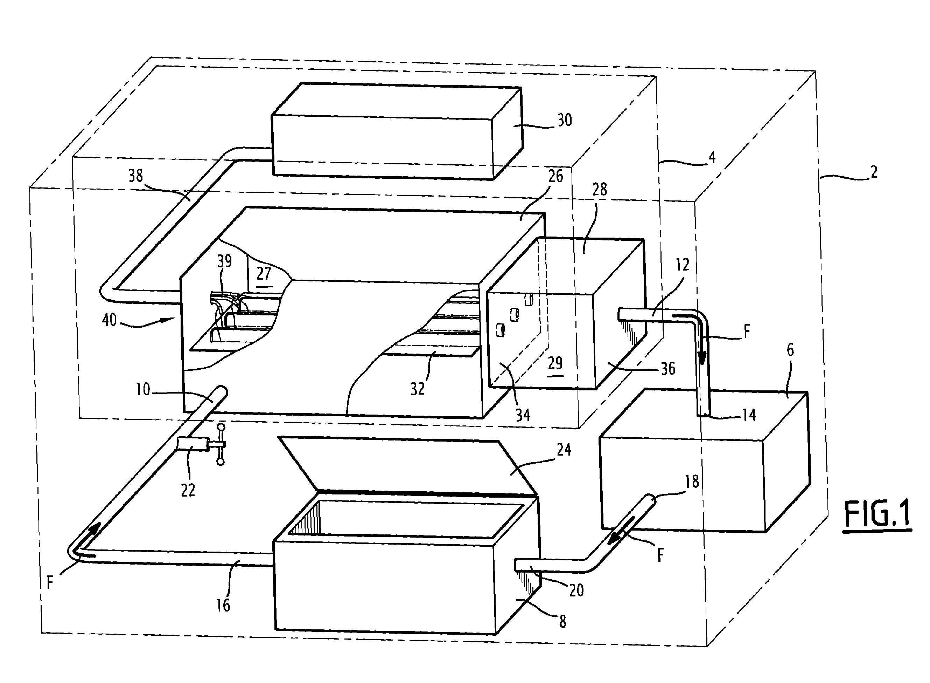

La figure 1 montre une installation 2 de traitement par fluide délimitée par un parallélépipède en lignes pointillées. En l'occurrence, le traitement est une stérilisation à la pression atmosphérique d'objets ou de denrées à l'aide d'air ozoné. L'air F circulant à l'intérieur de l'installation 2 est ozoné par une réaction plasmochimique.

L'installation 2 fonctionne de préférence en circuit fermé et comprend un dispositif 4 de production d'une espèce chimique, une pompe de fluide 6, et une enceinte de traitement 8. Le dispositif 4, à savoir un générateur d'ozone, dispose de moyens d'introduction de l'air F, à savoir d'une amenée 10, et de moyens d'extraction de l'air ozoné, à savoir d'un conduit d'extraction 12. Le conduit d'extraction 12 est relié à une entrée 14 de la pompe de fluide 6. La pompe 6 est de préférence une pompe mécanique à membrane. L'amenée 10 est reliée à une sortie 16 de l'enceinte de traitement 8. Une sortie 18 de la pompe de fluide 6 est reliée à une entrée 20 de l'enceinte de traitement 8. De manière préférée, l'amenée 10 comprend un moyen de réglage 22 du débit du fluide à l'intérieur de l'amenée 10. Le moyen de réglage 22 peut notamment être une vanne.

L'enceinte de traitement 8 se présente notamment sous la forme d'un boîtier de stérilisation, de préférence hermétique, avec un couvercle 24.

Le générateur d'ozone 4, délimité par un parallélépipède en lignes pointillées, comprend un boîtier 26 d'admission de l'air, un boîtier 28 d'évacuation de l'air ozoné, une source micro-ondes 30 et une structure résonante micro-ondes 32. Les deux boîtiers 26, 28 ont une face commune 34. Les deux boîtiers 26 et 28 sont rapportés l'un à l'autre en assurant l'étanchéité. Le boîtier d'admission 26 définit une chambre d'admission 27 et le boîtier d'évacuation 28 définit une chambre d'évacuation 29.

L'amenée 10 débouche dans la chambre d'admission 27. Le conduit d'extraction 12 est de préférence agencé au niveau d'une face 36 du boîtier d'évacuation 28 à l'opposé de la face commune 34.

Dans l'exemple montré à la figure 1, la source micro-ondes 30 est une unité séparée située à l'extérieur des boîtiers 26 et 28. De préférence, la source de micro-ondes 30 est apte à générer des micro-ondes d'une fréquence de 500 à 900 MHz, de préférence de 600 à 800 MHz, et de manière particulièrement préférée d'environ 630 MHz. Le choix d'une telle bande de fréquences est avantageux en ce qu'il permet de réaliser la source de micro-ondes 30 à l'aide d'amplificateurs à bas coûts développés pour les télécommunications. De préférence, la puissance délivrée par la source de micro-ondes 30 est de l'ordre de 2 à 5 Watts par élément de type antenne quart d'onde.

La structure résonante micro-ondes 32 est agencée à l'intérieur de la chambre d'admission 27.

Des moyens 38 de transmission de micro-ondes, notamment un faisceau de câbles coaxiaux ultra haute-fréquence 39, relie la source de micro-ondes 30 à la structure résonante micro-ondes 32. Plus précisément, le faisceau de câbles 38 traverse la face 40 du boîtier d'admission 26 située à l'opposé de la face commune 34 en maintenant l'étanchéité.

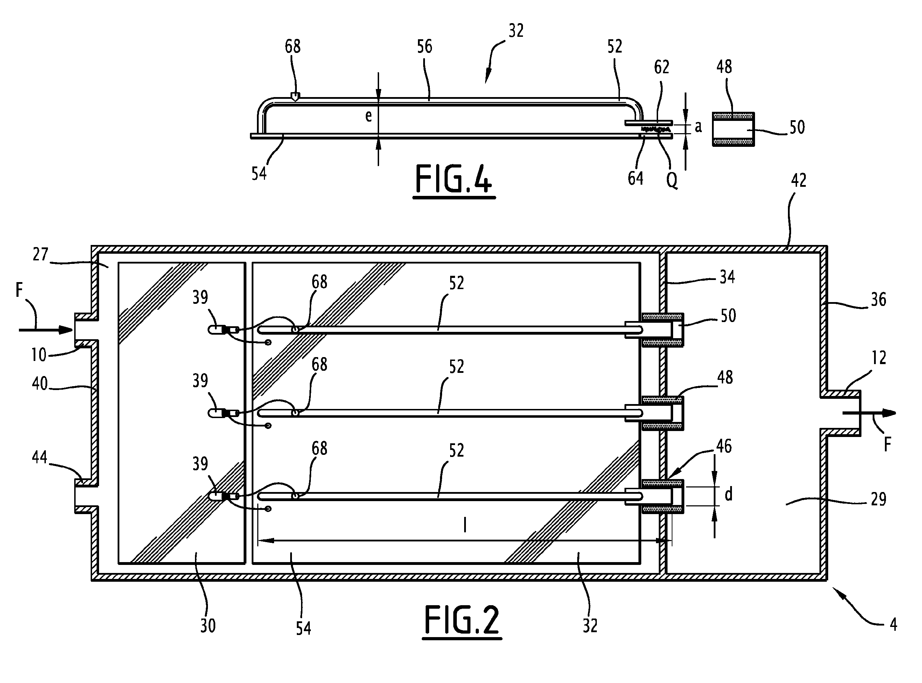

La figure 2 montre en coupe un générateur d'ozone 4 similaire à celui de la figure 1. Toutefois, dans le générateur d'ozone 4 selon la figure 2, la source de micro-ondes 30 est agencée à l'intérieur de la chambre d'admission 27. En outre, la chambre d'admission 27 et la chambre d'évacuation 29 sont délimitées par un boîtier commun 42. La chambre d'admission 27 est séparée de la chambre d'évacuation 29 par une paroi 34 en matière diélectrique telle que du polytétrafluoroéthylène (PTFE). L'amenée 10 et le conduit d'extraction 12 sont réalisés dans deux faces 36, 40 opposées du boîtier 42. La face 40 avec l'amenée 10 comprend en outre une ouverture 44 pour le passage de l'alimentation électrique de la source de micro-ondes 30.

La liaison fluidique entre la chambre d'admission 27 et la chambre d'évacuation 29 est assurée par trois trous 46 pratiqués dans la paroi 34. Chaque trou 46 accueille une bague ou gaine cylindrique 48, de préférence en céramique ou en alumine. Chaque bague 48 définit une chambre de réaction 50. Le diamètre intérieur d des bagues 48 est de préférence de l'ordre de 6 millimètres.

A la figure 2, la structure résonante micro-ondes 32 dispose de trois éléments de type antenne quart d'onde 52 et d'une surface de masse 54. La surface de masse 54 est notamment un plan de masse. Bien entendu, le nombre d'éléments de type antenne quart d'onde 52 peut varier en fonction du mode de réalisation de l'invention.

Un diélectrique est situé entre les éléments de type antenne quart d'onde 52 et la surface de masse 54. La fréquence de résonance de la structure résonante 32 est fonction du diélectrique utilisé. Le diélectrique est notamment de l'air.

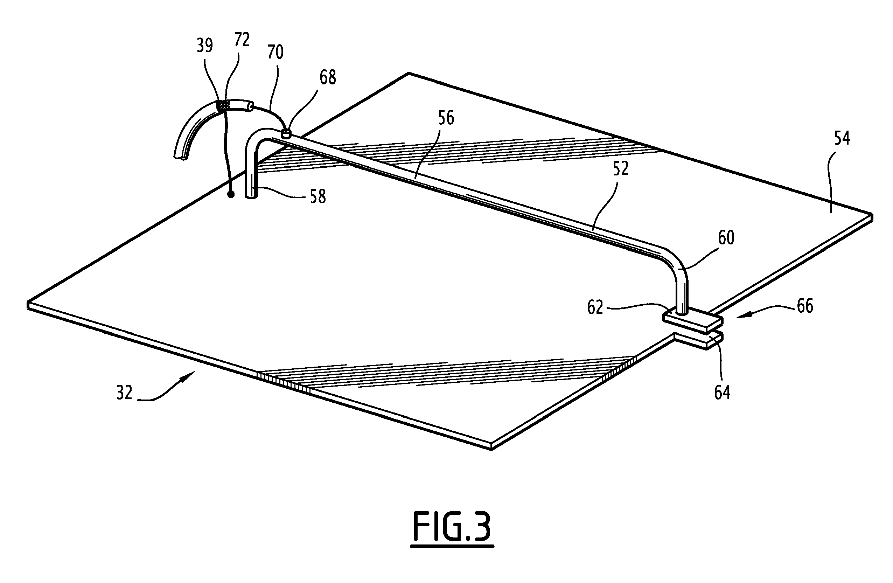

La figure 3 montre en perspective la structure résonante micro-ondes 32 du générateur d'ozone 4 de la figure 2. Par souci de simplicité, deux des trois éléments de type antenne quart d'onde 52 ont été omises. La figure 4 est une vue de côté de la structure résonante micro-ondes 32 de la figure 3.

L'élément de type antenne quart d'onde 52 comprend un tronçon principal 56 ainsi qu'une première 58 et deuxième 60 extrémité reliées au tronçon principal et sensiblement perpendiculaire à celui-ci. La première et deuxième extrémité 58 et 60 sont normales au plan de masse 54, tandis que le tronçon principal 56 est sensiblement parallèle au plan de masse 54. La première extrémité 58 de l'élément de type antenne quart d'onde 52 est reliée au plan de masse 54. La seconde extrémité 60 est libre et saillante vers le plan de masse 54 à partir du tronçon principal 56. La seconde extrémité libre 60 se termine en une électrode d'antenne 62 sensiblement parallèle au plan de masse 54. Le plan de masse 54 présente une électrode de masse 64 en regard de l'électrode d'antenne 62.

De préférence, les deux électrodes 62 et 64 se présentent sous forme de deux languettes en vis-à-vis et espacées de quelques millimètres. En particulier, l'électrode d'antenne 62 peut se présenter sous la forme d'une plaque avec une surface comprise entre 5 et 25 mm². De manière particulièrement préférée, la distance a entre les deux électrodes 62 et 64 est d'environ 3 millimètres, cf. la figure 4.

Les deux électrodes 62 et 64 constituent ensemble un générateur de plasma 66. Ce générateur de plasma 66 est inséré dans la chambre de réaction 50 définie par chaque bague 48. (cf la figure 4).

La distance e entre le tronçon principal 56 et le plan de masse 54 est de préférence de l'ordre de 6 à 8 millimètres. De préférence, la longueur l (cf la figure 2) de chaque élément de type antenne quart d'onde 52 est de l'ordre de 100 millimètres, la longueur l étant mesurée en allant du bord extrême de l'électrode d'antenne 62 à la zone de jonction entre le tronçon principal 56 et la première extrémité 58. La longueur I correspondant au quart de la longueur d'onde λ des micro-ondes, on a alors une longueur d'onde de travail de 100 X 4 = 400 millimètres ce qui correspondrait en rayonnement en espace libre (où la vitesse de phase c des ondes électromagnétiques est de l'ordre de 3 x 10⁸ m/s) à une fréquence de résonance f = c / λ d'environ 750 MHz. En pratique, la présence du plan de masse 54 change la vitesse de phase c et la structure résonante 32 résonne à une fréquence inférieure, en l'occurrence d'environ 630 MHz. Cette fréquence peut être calculée avec des outils numériques de simulation des structures électromagnétiques connus.

De préférence, la surface de masse 54 et les éléments de type antenne quart d'onde 52 sont réalisées en métal, les éléments de type antenne quart d'onde de préférence en inox, en tantale ou en platine, et la surface de masse de préférence en cuivre. Le choix du cuivre pour la surface de masse assure une bonne conductivité électrique surfacique impliquant un fort coefficient de surtension de la structure résonante.

Chaque élément de type antenne quart d'onde 52 dispose d'un point de connexion ultra haute-fréquence 68 au niveau du tronçon principal 56. L'emplacement exact du point de connexion 68 sur le tronçon principal 56 est choisi de façon à ce que l'impédance de l'élément de type antenne quart d'onde 52, vue de la source de micro-ondes 30, soit de l'ordre de 50 Ohms. Le point de connexion 68 est relié au conducteur central 70 d'un des câbles coaxiaux ultra haute-fréquence 39. Le blindage 72 du câble coaxial 39 est relié à la surface de masse 54.

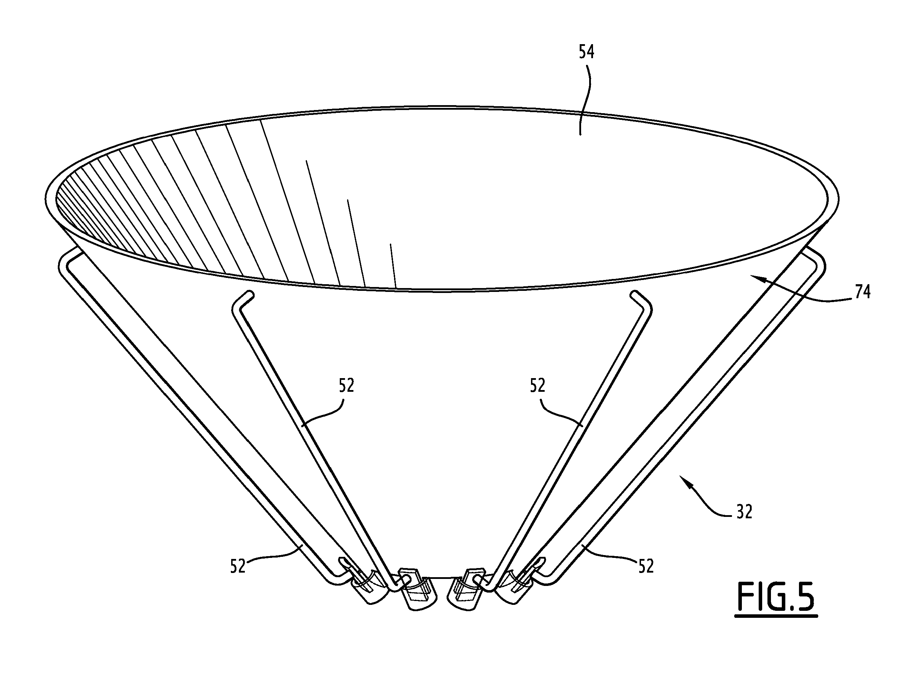

La figure 5 montre un deuxième mode de réalisation de la structure résonante micro-ondes 32. Dans ce mode de réalisation, la surface de masse 54 a la forme d'un tronc de cône. Les éléments de type antenne quart d'onde 52 sont réparties sur la circonférence 74 du tronc de cône et convergent vers la pointe imaginaire de celui-ci.

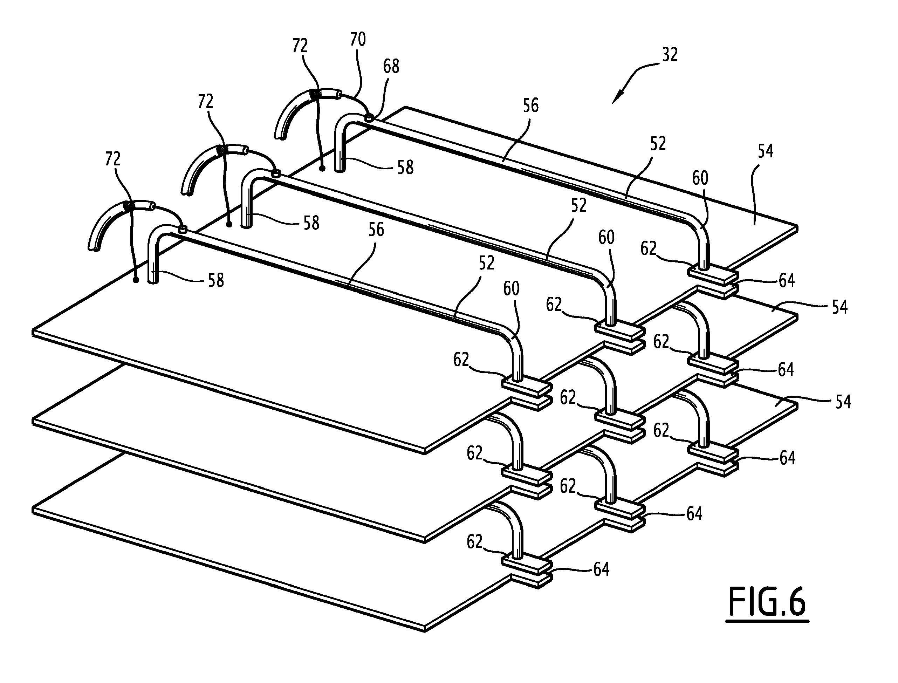

Le troisième mode de réalisation de la structure résonante micro-ondes 32 selon la figure 6 est un empilage de plusieurs structures résonantes micro-ondes selon la figure 3.

On va maintenant décrire le fonctionnement de l'installation de traitement 2 selon la figure 1. Tout d'abord, on ouvre le couvercle 24 du boîtier de stérilisation 8 et on y introduit un objet ou un liquide, tel que de l'eau non-potable, à stériliser. Le couvercle 24 est fermé. Ensuite, on lance la pompe à fluide 6 afin de faire circuler l'air F à l'intérieur du circuit fermé. La vanne 22 permet de régler le débit de l'air. Selon des essais réalisés sur un prototype, le débit de l'air est de préférence réglé à environ 1 litre/minute. L'ouverture de la vanne 22 est réglée de manière à obtenir une pression de travail à l'intérieur des chambres de réaction 50 inférieure à la pression atmosphérique et de préférence allant d'environ 10 à environ 50 KPa. La pression de travail à l'intérieur du boîtier de stérilisation 8 correspond sensiblement à la pression atmosphérique.

La stérilisation est lancée en allumant la source de micro-ondes 30 avec une fréquence d'environ 630 MHz et une puissance de quelques watts. Les micro-ondes générées par la source 30 sont acheminées vers la structure résonante micro-ondes 32 par l'intermédiaire du faisceau de câbles 38. Les micro-ondes sont injectées dans les éléments de type antenne quart d'onde 52 et la structure résonante micro-ondes 32 entre en résonance. Une dissipation d'énergie intervient dans le diélectrique situé entre les éléments de type antenne quart d'onde 52 et le plan de masse 54. Le champ électrique haute fréquence, de préférence de quelques centaines de volts par mètre, établi entre les électrodes d'antennes 62 et les électrodes de masse 64 crée un plasma local Q (cf la figure 4) dans l'espace parallélépipédique délimité par les électrodes 62 et 64. L'air entrant dans la chambre d'admission 27 par l'amenée 10 est acheminé vers les trous 46 réalisés dans la paroi 34. Les trous 46 étant la seule liaison fluidique entre la chambre d'admission 27 et la chambre d'évacuation 29, tout l'air est forcé à travers les chambres de réaction 50 contenant le plasma Q. Sous l'action du plasma Q, l'azote de l'air est excité et retourne à l'état non excité en émettant un fort rayonnement ultra-violet. Celui-ci excite et dissocie les molécules d'oxygène. On obtient ainsi des atomes d'oxygène qui se recombinent en ozone avec d'autres molécules oxygène. L'air enrichi en ozone quitte les chambres de réaction 50 vers la chambre d'évacuation 29 pour ensuite quitter la chambre d'évacuation 29 par le conduit d'extraction 12. L'air ozoné passe à travers la pompe 6 et atteint le boîtier de stérilisation 8. L'ozone de l'air stérilise alors l'objet contenu dans le boîtier de stérilisation. L'air ozoné quitte ensuite le boîtier de stérilisation 8 pour entrer à nouveau dans la chambre d'admission 27 par l'amenée 10. Cette circulation en boucle fermée permet de faire croître progressivement la concentration en ozone dans l'air F. Une concentration en ozone d'environ 100 ppm est obtenue en quelques minutes. Une fois la stérilisation achevée, l'objet stérilisé est retiré du boîtier de stérilisation 8.

On note que le fluide F circulant dans l'installation 2 est de préférence de l'air. En effet, l'air contient non seulement le réactif nécessaire à la production d'ozone, à savoir le dioxygène, mais aussi de l'azote qui catalyse la transformation du dioxygène en ozone. L'azote est un catalyseur dans le sens où il est excité par les décharges micro-ondes, et retourne par la suite à l'état non excité en émettant un rayonnement ultra-violet dissociant le dioxygène en atomes d'oxygène.

On pourrait choisir d'autres fluides que l'air pour la production d'ozone, il suffit que le fluide contienne de l'oxygène à l'état atomique et/ou moléculaire. Ainsi, l'utilisation de dioxygène pur est envisageable, néanmoins avec une production d'ozone plus faible en comparaison à l'utilisation d'air, par manque d'azote.

De manière plus générale, il est préférable d'utiliser comme fluide F un mélange comprenant du dioxygène et un moyen/catalyseur apte à dissocier le dioxygène en présence de micro-ondes.

L'installation de traitement 2 selon l'invention produit de l'ozone à partir de l'air ambiant à faible coût énergétique, sans utiliser de très haute tension comme dans les générateurs d'ozone à décharge couronne conventionnels. En outre, la production d'ozone du générateur d'ozone 4 selon l'invention n'est pratiquement pas perturbée par l'éventuelle présence d'humidité dans l'air utilisé. Il s'agit d'un avantage supplémentaire par rapport aux générateurs à décharge couronne. En effet, en présence d'humidité dans l'air à ozoniser la décharge couronne ne s'établit pas de façon satisfaisante.

Selon l'invention, l'ozone est généré par l'intermédiaire d'un plasma froid à pression atmosphérique réduite sur une structure résonante micro-ondes. Un avantage majeur de l'installation de traitement 2 selon l'invention est le traitement par plasma de la totalité de l'air circulant dans l'installation. En effet, tout l'air doit passer à travers les trous 46 de la face commune 34 et donc à travers le plasma Q. Le fort facteur de qualité de la structure résonante micro-ondes 32 permet la création d'un plasma avec une faible puissance micro-ondes. Avec un facteur de qualité au-dessus de 100, la puissance micro-ondes requise pour le dispositif selon l'invention est inférieure à 5 W pour obtenir un plasma présentant une densité entre 5·10¹⁶ et 10¹⁸ électrons par mètre cube, et de préférence de 10¹⁷ électrons par mètre cube.

La production d'ozone à des fins de stérilisation n'est qu'une parmi de nombreuses applications possibles de l'invention. On peut aussi penser à la production de liquides ozonés aux propriétés oxydantes utiles en médecine ou pour des traitements biologiques. Une version de plus forte puissance de l'installation 2 pourrait être utilisée comme moyen de traitement des eaux de piscines de faible volume.

La configuration de la structure résonante micro-ondes 32 en tronc de cône selon la figure 5 est particulièrement adaptée pour la réalisation de dépôts par plasma, et notamment pour la synthèse du diamant. Dans une telle application, la cible du dépôt est de préférence située sur l'axe du tronc de cône, à environ 2 centimètres de l'extrémité inférieure du tronc de cône, dans la chambre cible pompée. Des vitesses de dépôt importantes sont possibles grâce d'une part à la forte pression de travail, et d'autre part à la convergence sur la cible des gaz réactifs, ce qui n'est pas obtenu dans les réacteurs conventionnels à plasma.

Lorsque le fluide F utilisé est un mélange d'acétylène et d'ammoniac, il est possible de synthétiser des nanotubes de carbone, et plus généralement des nanomatériaux en utilisant d'autres mélanges, grâce à la structure résonante micro-ondes.

Le traitement d'échantillons biologiques est possible dans un mode de réalisation du dispositif 4 dans lequel la chambre d'évacuation 29 sert d'enceinte de traitement d'échantillons biologiques traités directement par le jet de fluide F ionisé et excité issu du plasma. Ce traitement est produit par l'interaction des radicaux libres à courte durée de vie en présence d'ozone ou d'autres espèces chimiques avec la surface biologique.

La structure résonante micro-ondes selon l'invention peut aussi être utilisée en synthèse chimique, par exemple pour la production d'oxygène sous forme excitée (oxygène singulet delta). L'oxygène singulet delta est utile notamment en chimie organique et est nécessaire en grande quantité pour la mise en oeuvre de l'inversion de population des lasers chimiques à iode de grande puissance.

Il est aussi envisageable d'utiliser la structure résonante micro-ondes selon l'invention pour la dissociation de molécules d'eau, notamment pour la production d'hydrogène, ou la dissociation de dioxyde de carbone.

Encore une autre application consiste à combiner le dispositif selon l'invention avec un système d'analyse spectroscopique en vue d'une détection de composés organiques volatils (COV). La structure résonante micro-ondes 32 ionise les composés organiques volatils qui sont ensuite analysés par le système d'analyse spectroscopique. L'analyse spectroscopique est réalisée soit dans la chambre de réaction 50, soit dans la chambre d'évacuation 29 dans le flux des ions générés après l'ionisation.

La structure résonante micro-ondes selon l'invention présente en outre l'avantage de pouvoir être mise en oeuvre de façon modulaire. Plusieurs structures résonantes micro-ondes peuvent être juxtaposées et/ou superposées, tel qu'illustré par la figure 6. Grâce à cette modularité, on peut réaliser une grande variété de générateurs plus ou moins compacts et avec une capacité de production adaptée.

## Revendications

1. Dispositif (4) de production d'une espèce chimique à partir d'un fluide (F) comprenant :
- une source de micro-ondes (30) ;
- une structure résonante micro-ondes (32) comportant un générateur (66) de plasma (Q) ;
- des moyens (38) de transmission des micro-ondes de la source de micro-ondes vers la structure résonante micro-ondes ;
- une chambre de réaction (50) accueillant le générateur de plasma ;
- des moyens (10) d'introduction du fluide (F) dans la chambre de réaction ; et
- des moyens (12) d'extraction de l'espèce chimique de la chambre de réaction,
la structure résonante micro-ondes (32) comprenant un élément résonant, notamment filaire, de type antenne quart d'onde (52),
**caractérisé en ce que** la structure résonante micro-ondes (32) comprend une surface de masse (54) et **en ce que** l'élément de type antenne quart d'onde (52) comprend un tronçon principal (56) sensiblement parallèle à la surface de masse (54).

2. Dispositif selon la revendication 1, dans lequel la surface de masse (54) est sous forme d'un plan ou d'un tronc de cône.

3. Dispositif selon l'une quelconque des revendications précédentes, dans lequel l'élément de type antenne quart d'onde (52) comprend une première (58) et deuxième (60) extrémités reliées au tronçon principal (56) et sensiblement perpendiculaires à celui-ci.

4. Dispositif selon la revendication 3, dans lequel la première extrémité (58) de l'élément de type antenne quart d'onde (52) est reliée à la surface de masse (54).

5. Dispositif selon la revendication 3 ou 4, dans lequel la seconde extrémité (60) est libre et saillante vers la surface de masse (54) à partir du tronçon principal (56).

6. Dispositif selon la revendication 5, dans lequel la seconde extrémité libre (60) présente une électrode d'antenne (62) sensiblement parallèle à la surface de masse (54), et dans lequel la surface de masse (54) présente une électrode de masse (64) en regard de l'électrode d'antenne (62), l'électrode d'antenne (62) et l'électrode de masse (64) constituant ensemble le générateur de plasma (66).

7. Dispositif selon la revendication 6, dans lequel la structure résonante micro-ondes (32) est adaptée pour créer un champ électrique, de préférence d'une intensité sensiblement comprise entre 200 et 1000 V/m, localisé entre l'électrode d'antenne (62) et l'électrode de masse (64) et pour ioniser un gaz présent entre l'électrode d'antenne (62) et l'électrode de masse (64).

8. Dispositif selon l'une quelconque des revendications précédentes, comprenant en outre une gaine cylindrique (48) en matière diélectrique définissant la chambre de réaction.

9. Dispositif selon l'une quelconque des revendications précédentes, comprenant en outre une chambre (27) d'admission du fluide accueillant la structure résonante micro-ondes, et une chambre (29) d'évacuation du fluide, la chambre de réaction assurant la liaison fluidique entre la chambre d'admission (27) et la chambre d'évacuation (29).

10. Dispositif selon l'une quelconque des revendications précédentes, le dispositif étant un générateur d'ozone.

11. Dispositif selon l'une quelconque des revendications précédentes, la source de micro-ondes (30) étant apte à générer des micro-ondes d'une fréquence de 500 à 900 MHz, de préférence de 600 à 800 MHz, et de manière particulièrement préférée d'environ 630 MHz.

12. Installation (2) de traitement par fluide en circuit fermé, l'installation comprenant :
- un dispositif (4) selon l'une quelconque des revendications précédentes ;
- une pompe de fluide (6) dont l'entrée (14) est reliée aux moyens (12) d'extraction de l'espèce chimique ;
- une enceinte de traitement (8) dont l'entrée (20) est reliée à la sortie (18) de la pompe de fluide, et dont la sortie (16) est reliée aux moyens (10) d'introduction du fluide.

13. Procédé de production d'une espèce chimique à l'aide d'un dispositif selon l'une quelconque des revendications 1 à 11, comprenant l'étape consistant à établir une pression de travail P à l'intérieur de la chambre de réaction allant d'environ 10 à environ 50 kPa.

14. Utilisation d'un dispositif selon l'une quelconque des revendications 1 à 11 pour la production d'une espèce chimique à partir d'un fluide comprenant du dioxygène et un moyen apte à dissocier le dioxygène en atomes d'oxygène en présence de micro-ondes.

15. Utilisation selon la revendication 14, le fluide étant de l'air et le moyen de dissociation étant alors l'azote de l'air.
